# FASCICULE DE BREVET EUROPEEN

(11) **EP 2 651 408 B1**
(45) Date de publication et mention de la délivrance du brevet: **18.10.2017**
(21) Numéro de dépôt: 11811088.1
(22) Date de dépôt: 12.12.2011
(51) Int. Cl.: A61K 31/135, A61K 31/137, A61K 31/382, A61K 31/4245, A61K 31/661, A61P 31/18

(54) **AGONISTES DES RECEPTEURS S1P ET LEUR UTILISATION DANS LE TRAITEMENT DES INFECTIONS DU VIH**
S1P-REZEPTORAGONISTEN UND IHRE VERWENDUNG ZUR BEHANDLUNG VON HIV-INFEKTIONEN
S1P RECEPTOR AGONISTS AND USE THEREOF IN TREATING HIV INFECTIONS

(30) Priorité: 13.12.2010 FR 1060432
(43) Date de publication de la demande: 23.10.2013
(73) Titulaire: Centre National de la Recherche Scientifique (C.N.R.S.), 75794 Paris Cedex 16 (FR)
(72) Inventeur: FRANCOIS, Vincent, F-34080 Montpellier (FR); CORBEAU, Pierre, F-34090 Montpellier (FR); DUQUENNE, Charline, F-34090 Montpellier (FR)
(74) Mandataire: Cabinet Plasseraud
(86) Numéro de dépôt international: PCT/FR2011/052948
(87) Numéro de publication internationale: WO 2012/080641

(56) Documents cités:
- WO-A1-02/18395
- WO-A1-92/11004
- WO-A1-99/58526
- WO-A1-2008/092930
- WO-A2-2004/103279
- WO-A2-2009/074969
- KERSH ELLEN N ET AL: "Evaluation of the lymphocyte trafficking drug FTY720 in SHIVSF162P3-infected rhesus macaques.", THE JOURNAL OF ANTIMICROBIAL CHEMOTHERAPY APR 2009 LNKD- PUBMED:19218272, vol. 63, no. 4, avril 2009 (2009-04), pages 758-762, XP002630184, ISSN: 1460-2091
- IM DONG-SOON: "Pharmacological tools for lysophospholipid GPCRs: development of agonists and antagonists for LPA and S1P receptors.", ACTA PHARMACOLOGICA SINICA SEP 2010 LNKD- PUBMED:20729877, vol. 31, no. 9, septembre 2010 (2010-09), pages 1213-1222, XP009146354, ISSN: 1745-7254
- DONG-SOON IM ET AL: "Characterisation of the human and mouse sphingosine 1-phosphate receptor, s1p5 (EDG-8): STRUCTURE-ACTIVITY RELATIONSHIP OF SPHINGOSINE 1-PHOSPHATE RECEPTORS", BIOCHEMISTRY, AMERICAN CHEMICAL SOCIETY, US, vol. 40, no. 46, 1 janvier 2001 (2001-01-01), pages 14053-14060, XP002469448, ISSN: 0006-2960, DOI: 10.1021/BI011606I
- HANESSIAN ET AL: "Constrained azacyclic analogues of the immunomodulatory agent FTY720 as molecular probes for sphingosine 1-phosphate receptors", BIOORGANIC & MEDICINAL CHEMISTRY LETTERS, PERGAMON, ELSEVIER SCIENCE, GB, vol. 17, no. 2, 11 janvier 2007 (2007-01-11), pages 491-494, XP005827260, ISSN: 0960-894X, DOI: 10.1016/J.BMCL.2006.10.014
- ZHEN LI ET AL: "DISCOVERY OF POTENT 3,5-DIPHENYL-1,2,4-OXADIAZOLE SPHINGOSINE-1-PHOSPHATE-1 (S1P1) RECEPTOR AGONISTS WITH EXCEPTIONAL SELECTIVITY AGAINST S1P2 AND S1P3", JOURNAL OF MEDICINAL CHEMISTRY, AMERICAN CHEMICAL SOCIETY, US, vol. 48, no. 20, 6 octobre 2005 (2005-10-06), pages 6169-6173, XP002423058, ISSN: 0022-2623, DOI: 10.1021/JM0503244

## Description

### DOMAINE DE L'INVENTION

L'invention concerne des composés qui sont des agonistes d'un récepteur choisi parmi les récepteurs S1PR1, S1PR2, S1PR3, S1PR4 et S1PR5 pour une utilisation dans le traitement des infections à VIH.

### INTRODUCTION

Le virus de l'immunodéficience humaine (VIH) est un rétrovirus infectant l'homme et responsable du syndrome d'immunodéficience acquise (sida), qui est un état affaibli du système immunitaire le rendant vulnérable à de multiples infections opportunistes. Transmis par plusieurs fluides corporels : sang, sécrétions vaginales, sperme ou lait maternel, le sida est aujourd'hui considéré comme une pandémie ayant causé la mort d'environ 25 millions de personnes entre 1981 (date de la première identification de cas de sida) et janvier 2006. Il est estimé qu'environ 1 % des personnes âgées de 15 à 49 ans vivent avec le VIH, principalement en Afrique subsaharienne. Bien qu'il existe des traitements antirétroviraux luttant contre le VIH et retardant par conséquence l'apparition du sida, réduisant ainsi la mortalité et la morbidité, il n'existe à l'heure actuelle aucun vaccin ou traitement définitif.

Il y a donc un besoin pour de nouvelles molécules permettant de traiter efficacement les infections à VIH.

### RESUME DE L'INVENTION

Le VIH ou HIV pénètre dans une cellule cible par liaison de la protéine gp120 de l'enveloppe virale avec le récepteur CD4, liaison qui permet alors la formation d'un complexe ternaire avec le corécepteur CCR5 (virus à tropisme R5 ou VIH-R5). Ceci conduit à la fusion de l'enveloppe virale via la protéine gp41 avec la membrane cellulaire, ce qui permet l'entrée du virus. CCR5 est un récepteur de chimiokines présent sur différents types de lymphocytes, et appartient à la famille des récepteurs couplés aux protéines G (RCPG). CCR5 étant le principal corécepteur du VIH, il représente un intérêt physiopathologique particulier et une cible thérapeutique de choix.

Les inventeurs ont pu montrer que le récepteur S1PR1 s'hétérodimérise avec le corécepteur CCR5 et qu'il diminue l'infectivité du VIH. Les inventeurs ont également montré que les agonistes du récepteur S1PR1 peuvent diminuer l'infection des cellules par le VIH. A l'issue d'études longues et approfondies, les inventeurs ont également montré que les agonistes des récepteurs S1PR1, S1PR2, S1PR3, S1PR4 et S1PR5 sont susceptibles de diminuer l'infection par VIH.

Sans vouloir être liés par une théorie, les inventeurs envisagent plusieurs hypothèses pour expliquer le fonctionnement des agonistes selon l'invention.

Une première hypothèse est que l'agoniste, en se fixant sur un récepteur S1PR, favoriserait l'internalisation de l'hétérodimère S1PR1-CCR5, comme cela a été proposé pour le récepteur S1PR1 seul par Markus H. Gräler dans Cell Physiol Biochem 2010 ; 26 :79-86, pour expliquer l'immunomodulation induite par l'agoniste FTY720-P. Cette internalisation de l'hétérodimère S1PR1-CCR5 diminuerait par conséquent la quantité de corécepteur CCR5 à la surface de la cellule, expliquant ainsi la baisse de l'infection par le VIH.

Une deuxième hypothèse est que la stimulation d'un récepteur S1PR induite par la fixation de l'agoniste diminuerait la signalisation du récepteur CCR5. Ce phénomène, appelé hétérodésensibilisation, est bien connu. Ainsi, l'activation d'un récepteur S1PR, par son effet « inhibiteur » sur CCR5, pourrait influencer l'infection et la propagation du VIH. Un tel effet anti-VIH a notamment été documenté dans les cas de la stimulation de CXCR1 par l'interleukine 8 (Richardson et al., 2003. J Biol Chem 278:15867-15873), de celle du FPR par le peptide bactérien fMLF (Shen, W. et al., 2000. Blood 96:2887-2894), et de A2A par l'adénosine (Zhang et al., 2006. Blood 108:38-44).

Une troisième hypothèse est que la stimulation d'un récepteur S1PR induite par la fixation de l'agoniste modifierait l'interaction de CCR5 avec CD4 ou gp120, ce qui entrainerait une diminution de l'infection des cellules par le virus.

Enfin, à la lumière de résultats obtenus à l'aide de virus VIH-1 dont l'enveloppe est remplacée par celle du virus de la stomatite vésiculaire (VSVG) et donc qui pénètrent dans la cellule par endocytose et indépendamment de CCR5, les inventeurs ont émis une quatrième hypothèse. Cette dernière repose sur la possibilité que la signalisation induite par la liaison d'un agoniste à son récepteur S1PR stimulerait la voie de transduction de ce récepteur d'une manière qui interfère avec le cycle réplicatif du VIH par un mécanisme indépendant de CCR5.

L'invention concerne donc les composés FTY720 et FTY720-P qui sont des agonistes du récepteur S1PR1 pour une utilisation dans des méthodes de traitement d'infections à VIH chez l'homme.

### DESCRIPTION DETAILLEE DE L'INVENTION

On entend par "agoniste du récepteur du sphingosine-1-phosphate" ou agoniste d'un récepteur S1PR", un composé qui se lie à au moins un récepteur du sphingosine-1-phosphate choisi parmi S1PR1, S1PR2, S1PR3, S1PR4, S1PR5.

Plus précisément, on entend par « agoniste du récepteur S1PR1 » un composé qui se lie au récepteur S1PR1 et qui, par exemple, entraîne l'internalisation du récepteur, et/ou la dissociation de l'hétérotrimère intracellulaire de protéines G en Gα-GTP et Gβγ, et/ou une augmentation de la phosphorylation du récepteur, et/ou l'activation de la voie de signalisation du récepteur. Ce terme englobe les agonistes dits « complets » ainsi que les agonistes dits « partiels » du récepteur du sphingosine-1-phosphate.

Dans la suite, les expressions "récepteur S1PR1" et "récepteur S1P1" sont utilisées indifféremment.

Selon l'invention, on entend par le terme « traitement » ou « traiter », l'action de supprimer, réduire, inhiber la progression, ou prévenir l'apparition de la condition ou de la maladie à laquelle ce terme s'applique ; ou l'action de supprimer, réduire, inhiber la progression, ou prévenir l'apparition d'un ou plusieurs symptômes de la condition ou de la maladie à laquelle ce terme s'applique.

Selon l'invention, on entend par « infection à VIH » ou « infection à HIV » une infection par n'importe quel type, groupe ou clade de virus de l'immunodéficience humaine (VIH). En effet, le VIH est un virus qui possède une très importante variabilité génétique et présente ainsi une très grande diversité. Le VIH peut être de deux types : VIH-1, le plus présent dans le monde, ou VIH-2, moins pathogène que VIH-1. Il comprend le VIH-2A et le VIH-2B. Au sein de chaque type existent plusieurs groupes qui, à leur tour, comportent des clades. Le VIH-1 est classé en quatre groupes : groupe M (pour major group), groupe O (pour outlier group), groupe N (pour non-M, non-O group) et groupe P. En 2005, le groupe M prédominait largement avec plus de 40 millions de personnes contaminées, contre un peu plus de 500 pour le groupe O et seulement 7 pour le groupe N. Le groupe M comprend neuf sous-types ou clades (de A à D, de F à H, J et enfin K). S'ajoutent plusieurs formes recombinantes (en anglais « circulating recombinant form » ou CRF), qui ont pour origine la multiple infection d'une cellule par des sous-types différents, ce qui entraîne des mélanges dans le génome viral.

### Agonistes des récepteurs du sphingosine-1-phosphate (S1PR)

La description concerne un composé qui est un agoniste d'au moins un récepteur du sphingosine-1-phosphate pour une utilisation dans des méthodes de traitement d'infections à VIH chez l'homme ou l'animal. L'invention concerne un composé qui est un agoniste d'au moins un récepteur choisi parmi les récepteurs S1PR1, S1PR2, S1PR3, S1PR4 et S1PR5 pour une utilisation dans une méthode de traitement d'une infection à VIH chez l'homme, caractérisé en ce que ledit composé est FTY720 ou FTY720-P.

Le S1PR1 (ou S1P1) a une séquence de 382 acides aminés, accessible sous le numéro d'accession NP_001391.2 sur la base NCBI. Cette séquence est représentée par SEQ ID N°1.

Le S1PR2 (ou S1P2) a une séquence de 353 acides aminés accessible sous le numéro d'accession NP_001391 sur la base NCBI. Cette séquence est représentée par SEQ ID N°2. Le S1PR3 (ou S1P3) a une séquence de 378 acides aminés accessible sous le numéro d'accession NP_005217 sur la base NCBI. Cette séquence est représentée par SEQ ID N°3. Le S1PR4 (ou S1P4) a une séquence de 384 acides aminés accessible sous le numéro d'accession NP_003766 sur la base NCBI. Cette séquence est représentée par SEQ ID N°4. Le S1PR5 (ou S1P5) est présent sous forme de deux isoformes.

La première isoforme a une séquence de 310 acides aminés accessible sous le numéro d'accession AAH67781 sur la base NCBI. Cette séquence est représentée par SEQ ID N°5. La seconde isoforme a une séquence de 398 acides aminés accessible sous le numéro d'accession NP_001159687 sur la base NCBI. Cette séquence est représentée par SEQ ID N°6.

Les agonistes des récepteurs du sphingosine-1-phosphate (S1PR) sont connus de l'art antérieur et l'homme du métier dispose des connaissances générales suffisantes pour identifier des composés agonistes d'un récepteur S1PR. Pour ce faire, il peut mettre en oeuvre un test fonctionnel de liaison de ³⁵S-GTPγS in vitro. Il peut par exemple se référer à la publication DS. Im et al., Mol. Pharmacol. 2000 ; 57-753. Dans ce test fonctionnel, la liaison entre le GTPγS et les protéines G médiée par le ligand est mesurée dans un tampon de liaison du GTP (en mM : 50 HEPES ; 100 NaCl, 10 MgCl₂, pH 7,5) en utilisant 25 µg d'une membrane préparée à partir de cellules HEK293 transfectées transitoirement. Le ligand est ajouté aux membranes en présence de 10 µM de GDP et de 0,1 nM de ³⁵S-GTPγS (1200 Ci/mmol) et incubé à 30°C pendant 30 minutes. Le GTPγS lié est séparé du GTPγS non-lié par un collecteur Brandel (Gaithersburg, MD), et compté avec un compteur de scintillant liquide. Typiquement, le composé selon l'invention est choisi dans le groupe comprenant la sphingosine-1-phosphate, FTY720, FTY720-P, AUY954, CYM-5442, CYM-5181, SEW2871, VPC01091, DS-SG-44, KRP-203-P, DihydroS1P, Composé 26, Composé 12, la sphingosylphosphorylcholine et AFD-R.

Il est également décrit un composé qui est un agoniste d'au moins deux récepteurs choisis parmi les récepteurs S1PR1, S1PR2, S1PR3, S1PR4 et S1PR5 pour une utilisation dans une méthode de traitement d'une infection à VIH chez l'homme ou l'animal. Typiquement, ledit composé est un agoniste d'au moins :
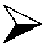 des récepteurs S1PR1 et S1PR2; ou
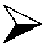 des récepteurs S1PR1 et S1PR3; ou
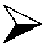 des récepteurs S1PR1 et S1PR4; ou
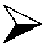 des récepteurs S1PR1 et S1PR5; ou
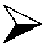 des récepteurs S 1 PR2 et S1PR3; ou
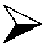 des récepteurs S 1 PR2 et S1PR4; ou
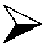 des récepteurs S 1 PR2 et S1PR5; ou
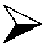 des récepteurs S 1 PR3 et S1PR4; ou
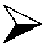 des récepteurs S 1 PR3 et S1PR5; ou
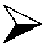 des récepteurs S 1 PR4 et S1PR5. Le composé est choisi parmi les composés cités dans le tableau ci-dessous:

| **COMPOSES** | **AGONISTE DE S1PR1** | **AGONISTE DE S1PR2** | **AGONISTE DE S1PR3** | **AGONISTE DE S1PR4** | **AGONISTE DE S1PR5** |
|---|---|---|---|---|---|
| sphingosine-1-phosphate | X | X | X | X | X |
| FTY720-P | X | | X | X | X |
| AUY954 | X | | X | | X |
| VPC01091 | X | | | X | X |
| DS-SG-44 | X | X | X | | |
| KRP-203-P | X | | | X | |
| DihydroS1P | X | X | X | X | X |
| composé 26 | X | | X | X | X |
| Composé 12 | | | | X | X |
| Sphingosylphosphorylcholine | X | X | X | X | X |
| AFD-R | X | | X | X | X |

### Agonistes du S1PR1

Ledit agoniste est un agoniste du récepteur S1PR1. Typiquement, il s'agit d'un agoniste de bas poids moléculaire, par exemple une petite molécule organique (naturelle ou pas). Le terme « petite molécule organique » se réfère à une molécule, naturelle ou pas, d'une taille comparable à celle des molécules organiques généralement utilisées comme médicament. Ce terme exclu les macromolécules (par exemple les protéines, les molécules d'acide nucléique, etc.). Des petites molécules organiques préférées ont une taille d'au plus 10 000 Da, de préférence d'au plus 5 000 Da, plus préférentiellement d'au plus 2 000 Da, encore plus préférentiellement d'au plus 1 000 Da.

Les récepteurs du sphingosine-1-phosphate sont connus. Parmi ces récepteurs, le S1PR1 ou (« Sphingosine-1-phosphate receptor 1 ») est largement décrit dans la littérature. Il s'agit d'un récepteur appartenant à la famille des récepteurs couplés aux protéines G, dont le ligand naturel est la sphingosine-1-phosphate (S1P). S1PR1 est également connu sous les noms suivants: EDG1, S1P1, ECGF1, EDG-1, CHEDG1, D1S3362, FLJ58121. Il est exprimé de manière ubiquitaire et sa délétion génétique chez la souris a mis en évidence son rôle clé dans l'angiogenèse et dans la maturation vasculaire, ainsi que dans la régulation du recrutement des cellules immunitaires (Takabe et al., Pharmacol Rev 2008 June ; 60(2) :181-195). L'implication de la sphingosine-1-phosphate dans le cancer a également été décrite par Weng In Leong et al., dans Biochimie 92 (2010) 716-723.

Les agonistes du récepteur S1PR1 sont également largement décrits dans de nombreuses revues scientifiques. On peut citer notamment la revue de Dong-Soon IM publiée dans Acta Pharmacologica Sinica (2010) 31 :1213-1222. On peut également citer les demandes de brevet EP1905434A1 ou WO2010/075239A1 qui décrivent différentes classes d'agonistes.

Les agonistes du récepteur S1PR1 sont typiquement des analogues de la sphingosine, comme les dérivés de 2-substitué 2-amino-propane-1,3-diol ou les dérivés de 2-amino-propanol. Les agonistes du récepteur S1PR1 sont des composés comprenant typiquement un groupe de formule X 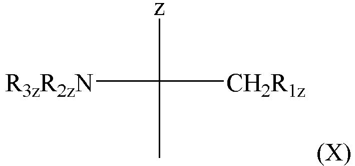 dans laquelle Z est H, C₁₋₆alkyle, C₂₋₆alcényle, C₂₋₆alcynyle, phényle, phényle substitué par OH, C₁₋₆alkyl substitué par 1 à 3 substituants choisis dans le groupe constitué de halogène, C₃₋₈cycloalkyle, phényle et phényle substitué par OH ; ou CH₂-P_{4z} où P_{4z} est OH, acyloxy ou un résidu de formule (a) 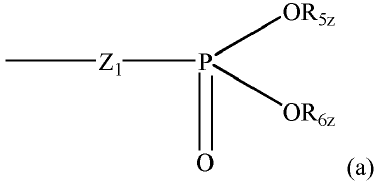
où Z₁ est une liaison directe ou O, de préférence O;
chacun de R_{5z} et R_{6z}, indépendamment, est H, ou C₁₋₄alkyle optionnellement substitué par 1, 2 ou 3 atomes d'halogène ;
R_{1z} est OH, acyloxy ou un résidu de formule (a) ; et chacun de R_{2z} et R_{3z}, indépendamment, est H, C₁₋₄alkyle ou acyle.

Le groupe de formule (X) est un groupe fonctionnel qui est attaché comme un groupe terminal à un groupe qui peut être hydrophile ou lipophile et peut comprendre un ou plusieurs résidus aliphatique, alicyclique, aromatique et/ou hétérocyclique. La molécule résultante fonctionne comme un agoniste d'un récepteur S1PR1.

De manière préférée, au moins un de Z et R_{1z} est ou comprend un résidu de formule (a).

Des exemples d'agonistes du récepteur S1PR1 comprennent :
(i) les composés décrits dans EP627406A1, par exemple un composé de formule I : 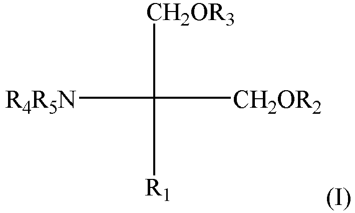
   où R₁ est une chaine C₁₂₋₂₂ linéaire ou ramifiée
      - qui peut avoir dans la chaîne une liaison ou un hétéroatome choisi parmi une liaison double, une liaison triple, O, S, NR₆, où R₆ est H, C₁₋₄ alkyle, aryl-C₁₋₄alkyle, acyle ou (C₁₋₄alkoxy)carbonyle, et carbonyle, et/ou
      - qui peut avoir comme substituant C₁₋₄alkoxy, C₂₋₄alcényloxy, C₂₋₄alcynyloxy, arylC₁₋₄alkyl-oxy, acyle, C₁₋₄alkylamino, C₁₋₄alkylthio, acylamino, (C₁₋₄alkoxy)carbonyle, (C₁₋₄alkoxy)-carbonylamino, acyloxy, (C₁₋₄alky)carbamoyle, nitro, halogène, amino, hydroxyimino, hydroxy ou carboxy;
   ou R₁ est
      - un phénylalkyle où l'alkyle est une chaine carbonée linéaire ou ramifiée en C₆₋₂₀ ; ou
      - un phénylalkyle où l'alkyle est une chaine carbonée linéaire ou ramifiée en C₁₋₃₀ où ledit phénylalkyle est substitué par
         ∘ une chaine carbonée linéaire ou ramifiée en C₆₋₂₀ optionnellement substituée par un halogène,
         ∘ une chaine alkoxy linéaire ou ramifiée en C₆₋₂₀ optionnellement substituée par un halogène,
         ∘ une chaine alcényloxy linéaire ou ramifiée en C₆₋₂₀,
         ∘ un phényl-C₁₋₁₄alkoxy, halophényl-C₁₋₄alkoxy, phényl-C₁₋₁₄alkoxy-C₁₋₁₄alkyl, phénoxy-C₁₋₄alkoxy or phénoxy-C₁₋₄alkyle,
         ∘ un cycloalkylalkyle substitué par un C₆₋₂₀alkyle,
         ∘ un hétéroarylalkyle substitué par un C₆₋₂₀alkyle,
         ∘ un C₆₋₂₀alkyle hétérocyclique, ou
         ∘ un alkyle hétérocyclique substitué par un C₂₋₂₀alkyle,
   et où le groupement alkyle peut avoir :
      - dans la chaîne carbonée, une liaison ou un hétéroatome choisi parmi une liaison double, une liaison triple, O, S, sulfinyle, sulfonyle, ou NR₆, où R₆ est tel que défini précédemment, et
      - comme substituant, C₁₋₄alkoxy, C₂₋₄alcényloxy, C₂₋₄alcynyloxy, arylC₁₋₄alkyl-oxy, acyle, C₁₋₄alkylamino, C₁₋₄alkylthio, acylamino, (C₁₋₄alkoxy)carbonyle, (C₁₋₄alkoxy)-carbonylamino, acyloxy, (C₁₋₄alky)carbamoyle, nitro, halogène, amino, hydroxy ou carboxy; et
   chacun de R₂, R₃, R₄ et R₅, indépendamment, est H, C₁₋₄ alkyle ou acyle,
   ou un sel ou un hydrate pharmaceutiquement acceptable de celui-ci.
(ii) les composés décrits dans WO 02/18395, par exemple un composé de formule IIa ou IIb 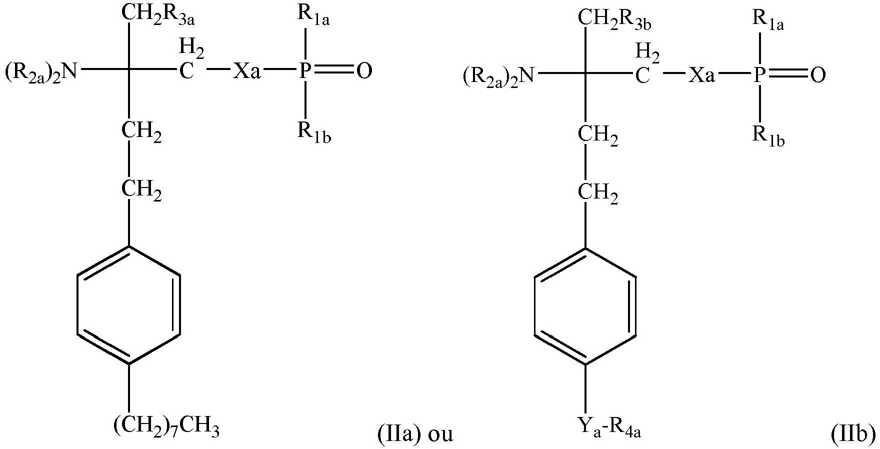
   où Xₐ est O, S, NR₁ₛ ou un groupe -(CH₂)ₙₐ-, ledit groupe étant substitué ou non par 1 à 4 halogènes ; nₐ est 1 ou 2, R₁ₛ est H ou (C₁₋₄)alkyle, ledit alkyle étant substitué ou non par un halogène, R₁ₐ est H, OH, (C₁₋₄)alkyle ou O(C₁₋₄)alkyle où l'alkyle est substitué ou non par 1 à 3 halogènes ; R_{1b} est H, OH ou (C₁₋₄)alkyle, où l'alkyle est substitué ou non par un halogène ; chaque R₂ₐ est indépendamment choisi parmi H ou (C₁₋₄)alkyle, ledit alkyle étant substitué ou non par un halogène ; R₃ₐ est H, OH, halogène ou O(C₁₋₄)alkyle où l'alkyle est substitué ou non par un halogène ; et R_{3b} est H, OH, halogène, (C₁₋₄)alkyle où l'alkyle est substitué ou non par hydroxyle, ou O(C₁₋₄)alkyle où l'alkyle est substitué ou non par un halogène; Yₐ est -CH₂-, -C(O)-, -CH(OH)-, -C(=NOH)-, O ou S, et R₄ₐ est (C₄₋₁₄)alkyl ou (C₄₋₁₄)alcényle ;
   ou un sel ou un hydrate pharmaceutiquement acceptable de celui-ci.

Lorsque les composés de formules I, IIa ou IIb possèdent un ou plusieurs centres asymétriques, la présente invention doit être comprise comme couvrant les différents isomères optiques, racémates, diastéréoisomères et leurs mixtures. Les composés de formule IIa ou IIb, lorsque l'atome de carbone portant le groupement amine est asymétrique, ont de préférence la configuration R pour cet atome de carbone.

Les composés de formules I, IIa ou IIb peuvent être sous forme libre ou sous forme de sel. Des exemples de sels pharmaceutiquement acceptables des composés de formules I, IIa ou IIb comprennent les sels d'acide inorganique, tels que hydrochloride, hydrobromide et sulfate ; les sels d'acide organique, tels que acétate, fumarate, maléate, benzoate, citrate, malate, méthanesulfonate et benzènesulfonate ; ou lorsque cela est approprié, les sels de métaux, tels que sodium, potassium, calcium, aluminium ; des sels d'amines, tels que triéthylamine ; et des sels d'acide aminé dibasique, tel que la lysine. Les composés et leurs sels selon l'invention couvrent également leurs formes hydrate et solvate.

Dans les définitions ci-dessus :
▪ acyle peut être un résidu Ry-CO- où Ry est C₁₋₆alkyle, C₃₋₆cycloalkyle, phényle or phényl-C₁₋₄alkyle,
▪ sauf si indiqué autrement, alkyle, alkoxy, alcényle ou alcynyle peuvent être linéaires ou ramifiés,
▪ aryle peut être phényle ou naphtyle, de préférence phényle,
▪ « groupe hétérocyclique » représente un groupe hétérocyclique de 5 à 7 chaînons, ayant 1 à 3 hétéroatomes choisis parmi S, O et N. Des exemples de tels groupes hétérocycliques comprennent les groupes hétéroaryles indiqués précédemment, et les composés hétérocycliques correspondant à des groupes hétéroaryles partiellement ou complètement hydrogénés, comme par exemple furyle, thienyle, pyrrolyle, azepinyle, pyrazolyle, imidazolyle, oxazolyle, isoxazolyle, thiazolyle, isothiazolyle, 1,2,3-oxadiazolyle, triazolyle, tetrazolyle, thiadiazolyle, pyranyle, pyridyle, pyridazinyle, pyrimidinyle, pyrazinyle, tetrahydropyranyle, morpholinyle, thiomorpholinyle, pyrrolidinyle, pyrrolyle, imidazolidinyle, pyrazolidinyle, piperidinyle, piperazinyle, oxazolidinyle, isoxazolidinyle, thiazolidinyle or pyrazolidinyle. Des groupes hétérocycliques préférés sont les groupes hétéroaryles à 5 ou 6 chaînons et les plus préférés sont les groupes morpholinyle, thiomorpholinyle or piperidinyle.

Lorsque la chaine carbonée comme R₁ est substituée dans les composés de formule I, cette chaine est de préférence substituée avec un halogène, nitro, amino, hydroxy ou carboxy. Quand la chaine carbonée est interrompue par un phénylène optionnellement substitué, la chaine carbonée est de préférence non substituée. Quand le groupement phénylène est substitué, il est de préférence substitué par un halogène, nitro, amino, méthoxy, hydroxy ou carboxy.

Des composés préférés de formule I sont ceux dans lesquels R₁ est C₁₃₋₂₀alkyle, optionnellement substitué par nitro, halogène, amino, hydroxy ou carboxy, et, plus préférentiellement, ceux où R₁ est un phénylalkyle substitué par une chaine C₆₋₁₄alkyle optionnellement substituée par un halogène et le groupement alkyle est un C₁₋₆alkyle optionnellement substitué par un hydroxy. Plus préférentiellement, R₁ est un phényl-C₁₋₆alkyle substitué sur le phényle par une chaîne C₆₋₁₄alkyle, linéaire ou ramifiée, de préférence linéaire. La chaine C₆₋₁₄alkyle peut être ortho, méta ou para, de préférence para. De préférence, chacun de R₂ à R₅ est H.

Un composé de formule I préféré est le 2-amino-2-tétradécyl-1,3-propanediol.

Un composé particulièrement préféré de formule I est le FTY720, c'est-à-dire le 2-amino-2-[2-(4-octylphényl)éthyl]propane-1,3-diol dans sa forme libre ou sous forme d'un de ses sels pharmaceutiquement acceptable, ou une pro-drogue de celui-ci. Le composé est FTY720 hydrochloride, comme indiqué ci après :

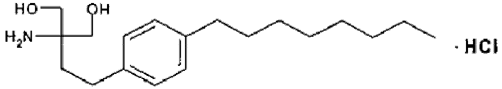

Un composé préféré de formule IIa est le FTY720-phosphate ou FTY720-P (R₂ₐ est H, R₃ₐ est OH, Xₐ est O, R₁ₐ et R_{1b} sont OH).

Un composé préféré de formule IIb est le un composé-phosphate dans lequel R₂ₐ est H, R_{3b} est OH, Xₐ est O, R₁ₐ et R_{1b} sont OH, Yₐ est O et R₄ₐ est heptyle. Le composé dans le groupe comprenant la sphingosine-1-phosphate, FTY720, FTY720-P, AUY954, CYM-5442, CYM-5181, SEW2871, VPC01091, DS-SG-44, KRP-203-P, DihydroS1P, Composé 26, Composé 12, la sphingosylphosphorylcholine et AFD-R. Ces composés sont notamment cités par Dong-Soon IM dans Acta Pharmacologica Sinica (2010) 31 :1213-1222.

Par "Composé 26", on entend le composé décrit dans la publication Li et al, "Discovery of potent 3,5-diphenyl-1,2,4-oxadiazole sphingosine-1-phosphate (SIP1) receptor agonists with exceptional selectivity against S1P2 and S1P3", Journal of Medicinal Chemistry, 2005 et ayant la formule suivante 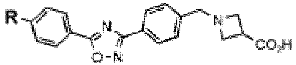 où R= (CH₃)₂ChCH₂-

Par "Composé 12", on entend le composé décrit dans la publication Hanessian et al, "Constrained azacyclic analogues of the immunomodulatory agent FTY720 as molecular probes for sphingosine 1-phosphate receptors", Bioorganic & Medicinal Chemistry Letters, 2007 et ayant la formule suivante:

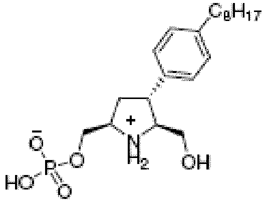

Par AFD-R, on entend le composé décrit dans la publication Brinkmann et al, "The immune modulator FTY720 targets sphingosine-1-phosphate receptors", The Journal of Biological Chemistry, 2002 et ayant la formule suivante:

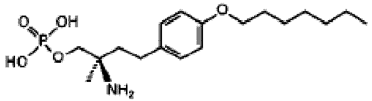

De manière préférée, le composé est un agoniste des récepteurs S1PR1, S1PR3, SPR4 et S1PR5. Le composé selon l'invention est FTY720 (également connu sous le nom « fingolimod ») ou FTY720-P, qui est la forme phosphorylée du FTY720. FTY720 et FTY720-P ont notamment été décrits par Mandala S. et al. dans Science 2002 ; 296 :346-9 et par Brinkmann V. et al. dans J Biol Chem 2002 ; 277 :21453-7.

Le FTY720 est un substrat de la sphingosine kinase 2 et est, sous sa forme phosphorylée, un agoniste des récepteurs S1PR1, S 1 PR3, S 1 PR4 et S1PR5.

Le FTY720-P est particulièrement pertinent pour une utilisation dans le traitement d'une infection à VIH. Cela s'explique notamment du fait de la synergie des effets du FTY720-P sur les récepteurs S1PR1, S1PR3, S1PR4 et S1PR5. Les inventeurs ont montré l'effet de protection contre l'infection à VIH de ce composé sur des lignées cellulaires, des cellules primaires. Enfin, ils ont également montré l'effet de cette molécule *in vivo* sur des souris sur lesquelles des lymphocytes humains ont été greffés avant d'être infectés par le VIH. En outre, les inventeurs ont montré que le FTY720-P permettait une diminution de l'infection à VIH chez des cellules n'exprimant pas le S1PR1. Ce résultat confirme que les récepteurs S1PR3 et/ou S1PR4 et/ou SPR5 ont également un rôle primordial dans la protection contre l'infection à VIH. Les agonistes sont des agonistes spécifiques du récepteur S1PR1 et d'au moins un récepteur choisi parmi S1PR2, S1PR3, S1PR4, S1PR5.

### Méthodes de traitement

Il est également décrit des méthodes de traitement d'un sujet souffrant d'une infection à VIH comprenant l'étape d'administrer audit sujet une quantité thérapeutiquement efficace d'au moins un composé qui est un agoniste d'au moins un récepteur choisi parmi les récepteurs S1PR1, S1PR2, S1PR3, S1PR4 et S1PR5, et préférentiellement qui est un agoniste du récepteur S1PR1.

Les composés selon l'invention peuvent également être administrés sous la forme de compositions pharmaceutiques, telles que définies ci après.

Par « quantité thérapeutiquement efficace » on entend une quantité suffisante pour traiter et/ou prévenir l'infection à VIH.

### Compositions pharmaceutiques

Les composés selon l'invention peuvent également être utilisés pour préparer des compositions pharmaceutiques pour le traitement d'infections à VIH.

Ainsi, il est également décrit des compositions pharmaceutiques pour une utilisation dans des méthodes de traitement d'infections à VIH chez l'homme ou l'animal, lesdites compositions comprenant au moins un composé selon l'invention et un véhicule pharmaceutiquement acceptable.

N'importe quel composé selon l'invention peut être combiné à tout type de véhicule ou excipient pharmaceutiquement acceptable, et optionnellement à une matrice à diffusion prolongée, telle qu'un polymère biodégradable, pour former une composition pharmaceutique selon l'invention.

Les termes « pharmaceutiquement acceptable » se réfèrent à des entités moléculaires et des compositions qui ne produisent pas de réaction inverse, allergique ou autre réaction non désirée lorsqu'elles sont administrées à un mammifère, particulièrement un humain. Un véhicule ou excipient pharmaceutiquement acceptable peut être solide, semi-solide ou liquide.

La forme des compositions pharmaceutiques, leur voie d'administration, leur dosage et leur posologie dépendent naturellement de la sévérité de l'infection, de son stade d'évolution, de l'âge, du sexe, du poids du sujet à traiter, etc.

Les compositions pharmaceutiques selon l'invention peuvent être formulées pour une administration topique, orale, intra-nasale, parentérale, intraveineuse, intramusculaire, sous-cutanée, ou autre.

Selon un mode de réalisation de l'invention, ladite infection à VIH est une infection à VIH-1. Selon un autre mode de réalisation, ladite infection à VIH étant une infection à VIH-2.

Les exemples suivants sont donnés à titre illustratif seulement, et ne sauraient limiter la portée de l'invention.

### LEGENDES DES FIGURES

**Figure 1****: Mesure de l'infectabilité des lignées HOS CCR5 LacZ et HOS CCR5 S1PR1 par le virus Ad8Luc.**
   L'infectabilité est mesurée par l'activité luciférase. Cette figure montre que la présence du récepteur S1PR1 inhibe l'infection de la lignée HOS par le virus VIH.
**Figure 2****: Effet du FTY720-P sur une lignée cellulaire HOS CCR5 S1PR1.**
   L'infectabilité est mesurée par l'activité luciférase. Cette figure montre que la pré-incubation avec FTY720-P des cellules exprimant S1PR1 diminue l'infection.
**Figure 3****: Mesure de l'infectabilité sur une lignée HOS CCR5 n'exprimant pas S1PR1.**
   Des cellules HOS CCR5 lacZ ont été préincubées pendant 1 heure, avec :
   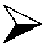 un agoniste spécifique de S1PR1, le SEW2871, ou
   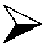 l'agoniste naturel de tous les récepteurs S1PR (S1PR1 à S1PR5), la sphingosine-1-phosphate (S1P), ou
   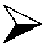 un agoniste de S1PR1, S1PR3, S1PR4 et S1PR5, le FTY720-P.

   Aucune différence significative n'est observée avec le SEW2871 tandis que le S1P et le FTY720-P permettent une diminution d'environ 50% de l'infection à VIH.

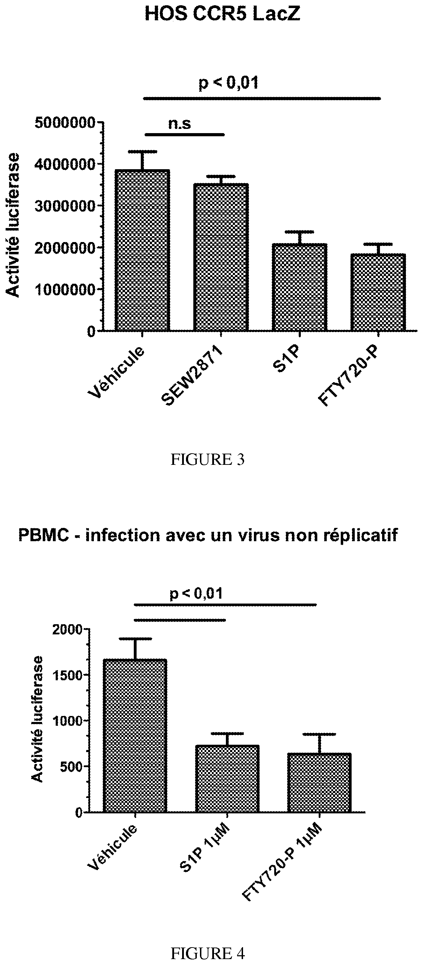

**Figure 4** **: Inhibition de l'infection de cellules primaires lymphocytaires par le FTY720-P.**
   Cette figure montre l'effet du S1PR1 et du FTY720-P sur des cellules mononuclées du sang périphérique (PBMC).
   Cette figure confirme l'effet du FTY720-P sur des cellules primaires.

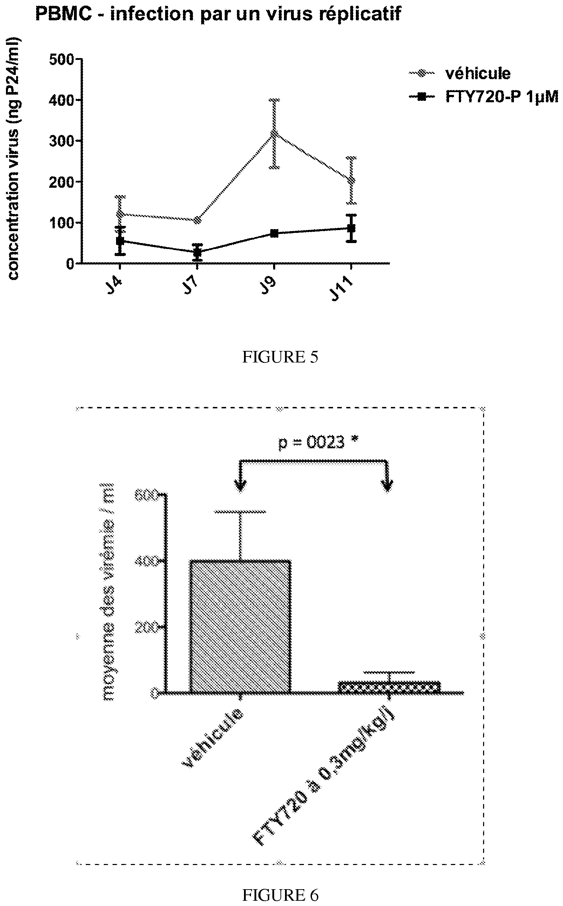

**Figure 5** **: Mesure de la production virale dans le milieu de culture de cellules primaires infectées avec un virus réplicatif Ad8 et préincubées avec du FTY720-P.**
   Cette figure montre une nette inhibition de la production virale par les PBMC.
**Figure 6****: Effet de FTY720 *in vivo***
   Cette figure montre une nette diminution de la virémie chez des souris greffées avec des cellules lymphocytaires humaines et infectés au VIH. Cette figure confirme l'effet in vivo du FTY720 sur la prévention de l'infection à VIH.

### EXEMPLES

### MATERIEL ET METHODES

Les matériels et méthodes utilisés pour l'ensemble de la partie expérimentale sont détaillés ci après.

### Cellules

Les lignées HOS-CD4 (AIDS Reagent Program, Rockvill, MD) et HEK-293T (simian virus 40 T antigen-transformed human embryonic kidney 293T, Genethon) sont cultivées en milieu DMEM supplémenté avec 10% de SVF (sérum de veau foetal), 10mM glutamax-1, 100 U/ml de pénicilline et 100 µg/ml de streptomycine. La lignée HEK-293T CD4+ CCR5+ (don de Martine Biard-Piechaczyk, Institut de Biologie, Montpellier, France) a été obtenue par transfection des gènes CD4 et CCR5 à partir de la lignée HEK-293T.

Les PBMC humaines en culture sont isolées à partir de sang de donneurs sains par centrifugation de densité sur milieu de séparation des lymphocytes (Eurobio) et cultivées en milieu RPMI supplémenté comme le DMEM à 37°C, 5% de CO2.

### Effet de S1PR1 sur l'infection de la lignée HEK-293T

Pour tester l'influence de l'expression de S1PR1 sur l'infection par HIV-1, 50 000 cellules de HEK-293T CD4+ CCR5+ ont été transfectées transitoirement par lipofection en les incubant pendant 24h avec un mélange de 50 ng de plasmide CMV-S1PR1, 20 ng du plasmide pRL-TK-Renilla Luciferase (Promega) et 0,5 µl de lipofectamine (Lipofectamine™ 2000, Invitrogen) dans 150 µl de milieu DMEM 10% SVF en plaque 96 puits, préalablement incubée avec une solution de D-polylysine pendant 30 min. Le clone plasmidique de S1PR1 a été obtenu auprès du Missouri S&T cDNA Resource Center (www.cdna.org). Un fragment portant S1PR1 a été cloné sous contrôle du promoteur CMV dans le vecteur d'expression pcDNA3.1+ (invitrogen). 24h après la transfection, les puits sont lavés au DMEM (Bio Whittaker), puis les cellules sont infectées avec 40 ng du virion Ad8-luc dans 180 µl de milieu de culture. Le virion Ad8-luc est un virus non réplicatif pseudotypé R5 obtenu par co-transfection de cellules HEK293T avec le plasmide de transfert pNL4.3 Luc.R-E- qui porte un gène viral *env* défectif ainsi que le gène de la luciférase Firefly inséré dans le gène viral *nef* (AIDS Reagent Program), et le plasmide pCMV-AD8-env qui code pour l'enveloppe R5 du prototype AD8 HIV-1 (Cho, Shibata & Martin (1996) J. Virol. 70, 7318-7321). 24h après l'infection, les cellules sont lavées une fois avec du milieu de culture, et remises en culture avec 200 µl de DMEM, 10% SVF. 40h à 48h après l'infection, les cellules sont lavées une fois au PBS, lysées avec 50 µl de tampon puis les activités luciférase Firefly et Renilla sont mesurées séquentiellement dans un luminomètre à l'aide du système Dual-Luciferase^{®} Reporter Assay (Promega, cat: E1910). L'activité luciférase Renilla est utilisée comme mesure du niveau de métabolisme cellulaire.

### Transduction des cellules HOS pour l'expression de CCR5 et S1PR1

Pour produire des vecteurs HIV exprimant les gènes CCR5, lacZ et S1PR1, les plasmides pWPXL-CCR5 (Desmetz et al., 2007, Clin Immunol 123, 148-154), pHRCMV-lacZ (Naldini et al., 1996, Science 272, 263-267) et pWPXL-S1PR1 ont été cotransfectés avec le plasmide packaging p8.2 et le plasmide pMD2G qui code pour l'enveloppe du virus de la stomatite vésiculaire dans des cellules 293T comme décrit précédemment (Lin et al., 2002, PNAS 99, 15590-15595). Pour construire le plasmide pWPXL-S1PR1, la séquence codante de S1PR1 a été amplifié par PCR à partir du plasmide pCDNA3.1-S1PR1 puis le fragment BamH1-Spe1 obtenu cloné dans le vecteur lentiviral pWPXL (addgene.org). Les cellules HOS ont été transduites (Lin *et al.,* 2002) d'abord avec le vecteur HIV-CCR5 et la lignée obtenue a été retransduite avec le vecteur HIV-S1PR1 ou le vecteur HIV-lacZ avec des quantités égales de virus en équivalent p24. L'expression membranaire des récepteurs CCR5 et S1PR1 a été évaluée en cytométrie de flux (FACScalibur, BDBiosciences) après marquage des cellules avec les anticorps monoclonaux de souris anti-human CD 195 (BD Pharmingen) et anti-human EDG1 (R&D), respectivement.

### Tests d'infection des cellules HOS avec le virus non réplicatif Ad8-luc

50 000 cellules HOS sont cultivées en triplicatas en plaque 96 puits, puis infectées avec 50 ng de virus Ad8-luc. 24h après l'infection, les cellules sont lavées deux fois avec du PBS, et remises en culture pendant 48h. Les cellules sont alors lavées une fois au PBS, lysées avec 50 µl de tampon, puis l'activité luciferase Firefly est mesurée dans un luminomètre à l'aide du kit Promega (Luciferase Assay System) .

### Tests d'infection des PBMC avec le virus réplicatif Ad8

Les PBMC sont activées par incubation pendant 72h dans du milieu de culture additionné de phytohémagglutinine (PHA, 1µg/ml) et d'interleukine 2 (IL2, 100 U/ml), puis après un lavage au RPMI, incubées en plaques 96 puits en triplicatas à 200 000 cellules dans 200 µl par puits. Elles sont ensuite infectées avec 70 ng d'équivalent p24 du prototype Ad8 HIV-1 pendant 18h, puis lavées 2X au PBS et incubées pendant 11 jours en ajustant le nombre de cellules à chaque prélèvement du surnageant de culture à J4, J7 et J9. La production de virus est suivie dans le surnageant de culture par mesure de la concentration de la protéine gag p24 par ELISA au moyen d'un kit commercial (Innotest HIV AG MAB, Ingen,réf:80563).

### Tests des molécules pharmacologiques

Pour les cellules HEK-293T CD4+ CCR5+, 24h après transfection par le plasmide S1PR1 ou par le vecteur vide, les cellules ont été incubées pendant 30 min puis infectées avec l'anticorps anti-CCR5 à 10 µg/ml (clone 2D7, Pharmingen, ref. 555991), ou avec MIP-1β à 100 ng/ml (R&D systems, ref. 271-BME) ou avec (*S*)-FTY720 Phosphate (FTY720-P) à 80 ng/ml (Echelon, ref. B-0721).

Pour les cellules HOS et les PBMC, les molécules S1P (Enzo, vendu par covalab ref. SL-140), FTY720-P (Echelon) ou SEW2871 (Cayman, ref. 10006440) ont été ajoutées dans le milieu de culture 1 heure avant l'infection aux concentrations indiquées.

### Modèle animal de souris immunologiquement humanisées

Les souris SCID sont de génotype cb17/Icr-Prkdc^{scid}/Crl. Ces souris immunodéprimées sont hébergées dans une animalerie A3/L3 dans des cages avec couvercle à filtre dans un portoir ventilé. Après une semaine d'acclimatation, les animaux sont reconstitués par injection intra-péritonéale de 30.10⁶ PBMC obtenues par centrifugation de densité sur les leucocytes d'un anneau de cytaphérèse non qualifié d'un donneur sain volontaire. La reconstitution est évaluée au 13^{ème} jour par dosage des Immunoglobulines (Ig) humaines présentes dans le sérum des souris par test Elisa à l'aide de l'anticorps anti-human whole Ig-PO, (MP Biomedical, cat n°55230). Les souris dont la concentration en Ig totale est supérieure à 100 µg/ml sont conservées pour l'expérience, et sont infectées 14 jours après la reconstitution avec la souche JR-CSF de HIV-1 (souche R5) à 1000 TCID50 dans 100µl. Un gavage journalier avec 100 µl de FTY720 (Cayman, vendu par Interchim ref. BM8045) dissout à 60µg/ml dans de l'eau distillée est débuté un jour avant l'infection et continué pendant 12 jours. Les souris non-traitées sont gavées avec le même volume d'eau distillée. 50 µl de sang est prélevé rétro-orbitalement et dilué avec 1 ml de plasma humain pour doser la charge virale au 6ème, 9ème et 12ème jour après l'infection après anesthésie à l'isoflurane. Les virémies sont évaluées par quantification de l'ARN viral dans le plasma des souris en qPCR avec le test AmpliPrep/COBAS TaqMan HIV-1 (Roche Diagnostics, ref. 05212294190). Les valeurs inférieures à 20 copies d'ARN/ml (soit 400 copies/ml de sérum de souris) étant inférieures au seuil de détection du kit sont considérées égales à zéro.

### Expériences de TR-FRET

La séquence codante de S1PR1 a été amplifiée par PCR à partir du 2ème codon jusqu'au codon stop entre des sites Mlu1 et Xba1, puis insérée à l'aide de ces sites de restriction dans les plasmides pRK5-HA-SNAP-mGlu2 et pRK5-FLAG-CLIP-mGlu2 (Doumazane, E. et al., FASEB J. 2010 Sep 27) en remplacement du gène mGlu2, de telle sorte que le récepteur S1PR1 est fusionné à l'extrémité N terminal soit avec un épitope de l'hémagglutinine (HA) et l'enzyme SNAP, soit avec un épitope FLAG et l'enzyme CLIP, insérés derrière un peptide signal.

Des cellules HEK293 ont été transfectées par lipofection avec de l'ADN des plasmides CLIP-CCR5 (7 ng pour 100 000 cellules) et SNAP-RCPG (gamme de 1 à 100 ng pour 100 000 cellules) et déposées à raison de 100 000 cellules par puits en plaque 96 puits noires pré-incubées avec une solution de polyornithine. 24 à 30h après la transfection, les cellules adhérentes sont lavées puis incubées avec du milieu de culture contenant les substrats des enzymes SNAP et CLIP couplés à des fluorophores pendant 2h à 37°. Les fluorophores sont du Lumi4®- Cryptate de Terbium lié à l'*O*⁶-benzylguanine (BG-Lumi4) utilisé à 0,3 µM, et de la fluorescéine liée à l'*O*²-benzylcytosine (BC-Fluorescéine) utilisé à 1 µM, substrats des enzymes SNAP et CLIP, respectivement. BG-Lumi4 et BC-Fluorescéine viennent de Cisbio Bioessays (Bagnols-sur-Cèze, France). Dans ces conditions, le marquage des récepteurs par les fluorophores est spécifique pour le SNAP ou le CLIP, total (100% des récepteurs sont marqués) et restreint aux récepteurs présents à la surface cellulaire (Doumazane, E. et al., FASEB J. 2010 Sep 27). De plus, les intensités de fluorescence du Lumi4 et de la fluorescéine sont proportionnelles au nombre de récepteurs présents à la surface cellulaire (Doumazane, E. et al., FASEB J. 2010 Sep 27). Les cellules sont ensuite lavées 4 fois avec du tampon Tris-Krebs à 37° (Maurel, D. et al., 2008, Nat Methods 5:561-567). La fluorescence et le TR-FRET sont mesurés dans 100 µl de tampon Tris-Krebs dans un spectrofluorimètre Infinite F500 (Tecan, Männedorf, Switzerland) avec les paramètres suivants: Lumi4 (excitation à 320 nm, émission à 620 nm, 150 µs de délai et 500 µs de temps d'intégration), fluorescéine (excitation à 485 nm, émission à 520 nm, 0 µs de délai et 1000 µs de temps d'intégration), trFRET (excitation à 320 nm, émission à 520 nm, 150 µs de délai et 500 µs de temps d'intégration). Le signal de FRET est corrigé par soustraction du FRET non spécifique obtenu après transfection du récepteur SNAP seul, et du FRET non spécifique obtenu avec le seul substrat BG-Lumi4 seul. Les valeurs de fluorescence du Lumi4 et de la fluorescéine sont corrigées par soustraction du signal non spécifique mesuré après transfection d'un plasmide vide.

### RESULTATS

### I. Effet des agonistes de l'invention sur des lignées HEK293T exprimant les récepteurs CD4 CCR5

### Etude de la capacité de S1PR1 à interférer avec l'infection par VIH

Le gène codant pour S1PR1 a été cloné dans un vecteur d'expression sous contrôle du promoteur CMV. Nous avons utilisé une lignée HEK293T exprimant les récepteurs CD4 CCR5 de manière constitutive et à un niveau suffisant pour permettre l'infection.

Les résultats ont montré que lorsqu'on introduit un plasmide permettant l'expression à la membrane du récepteur S1PR1 dans cette lignée, le niveau d'infection est abaissé d'un facteur deux environ par rapport au contrôle (plasmide vide). Comme attendu, l'apport d'un plasmide CCR5 qui augmente le niveau membranaire du co-récepteur du VIH a donné un niveau d'infection supérieur au contrôle. Dans ces expériences, il est contrôlé que l'effet sur l'infection des plasmides S1PR1 et CCR5 ne résulte pas d'un effet global sur le métabolisme cellulaire.

### Effet sur l'infection de la stimulation du récepteur S1PR1 par l'agoniste FTY720-P

Une fois la capacité de S1PR1 à interférer avec l'infection par VIH mise en évidence, nous avons testé l'effet d'un agoniste de S1PR1 sur l'infection à VIH, en utilisant la même méthode mais en incluant une étape d'incubation des cellules avec l'agoniste à tester juste avant l'infection.

Les résultats montrent qu'en présence du récepteur S1PR1 non stimulé, l'infection est diminuée de 60% par rapport au contrôle plasmide vide et qu'en présence du récepteur S1PR1 stimulé par l'agoniste FTY720-P pendant 30 min avant l'ajout du virus, l'infection est alors diminuée de 90% par rapport au contrôle plasmide vide. Cette diminution de l'infection avec FTY720-P est du même niveau que celle qui est obtenue dans cette expérience lorsque les cellules sont incubées dans les mêmes conditions avec un anticorps anti-CCR5 ou avec un ligand de CCR5 (MIP1-β), qui sont connus pour inhiber l'infection par le VIH (Wu, L. et al., 1997, J Exp Med 186 :1373-1381 ; Cocchi, F. et al., 1995, Science 270 (5243) :1811-1815).

### Mise en évidence d'une hétérodimérisation de S1PR1 avec CCR5 par la technique de HTRF

Les RCPG peuvent agir en tant qu'homodimères mais aussi par interactions hétérodimériques. Afin de préciser le mécanisme par lequel S1PR1 inhibe l'infection, nous l'avons testé pour sa capacité à hétérodimériser avec CCR5. Pour cela, nous avons utilisé une technologie de TR-FRET (Time Resolved- Fluorescence Resonance Energy Transfer), le HTRF (http://www.htrf.com/technology/). Dans cette technologie qui allie les principes du TRF (Time-Resolved Fluorescence) et du FRET (Fluorescence Resonance Energy Transfer), chacun des récepteurs CCR5 et S1PR1 est exprimé à la surface membranaire en fusion traductionnelle avec les enzymes SNAP et CLIP, respectivement, qui permettent la liaison spécifique et irréversible de deux fluorophores couplés à des substrats de ces enzymes. Un signal de FRET est détecté lorsque les 2 fluorophores sont à des distances compatibles avec une hétérodimérisation entre les récepteurs.

Les résultats ont montré que le signal de FRET obtenu entre les récepteurs S1PR1 et CCR5 est comparable à celui obtenu pour l'homodimère CCR5, ce qui atteste d'une interaction directe à la membrane entre CCR5 et S1PR1, probablement au sein d'un complexe hétérodimérique.

### II. Effet des agonistes de l'invention sur des lignées HOS-CD4

Les inventeurs ont corroboré leurs premiers résultats obtenus sur des lignées HEK293T exprimant les récepteurs CD4 CCR5 à l'aide de cellules HOS-CD4.

### L'expression de S1PR1 inhibe l'infection d'une lignée HOS par le VIH

Une lignée HOS (Human Osteo Sarcoma) exprimant le récepteur CD4 a été transduite par un vecteur lentiviral permettant l'expression constitutive du co-récepteur CCR5 à la membrane plasmique afin de la rendre infectable par le virus VIH-1. Cette lignée HOS CCR5 est transduite à nouveau pour obtenir l'expression à la membrane du récepteur S1PR1 (HOS CCR5 S1PR1). En parallèle, une lignée transduite avec le gène lacZ (HOS CCR5 lacZ) est obtenue par la même technique en contrôle d'infectabilité.

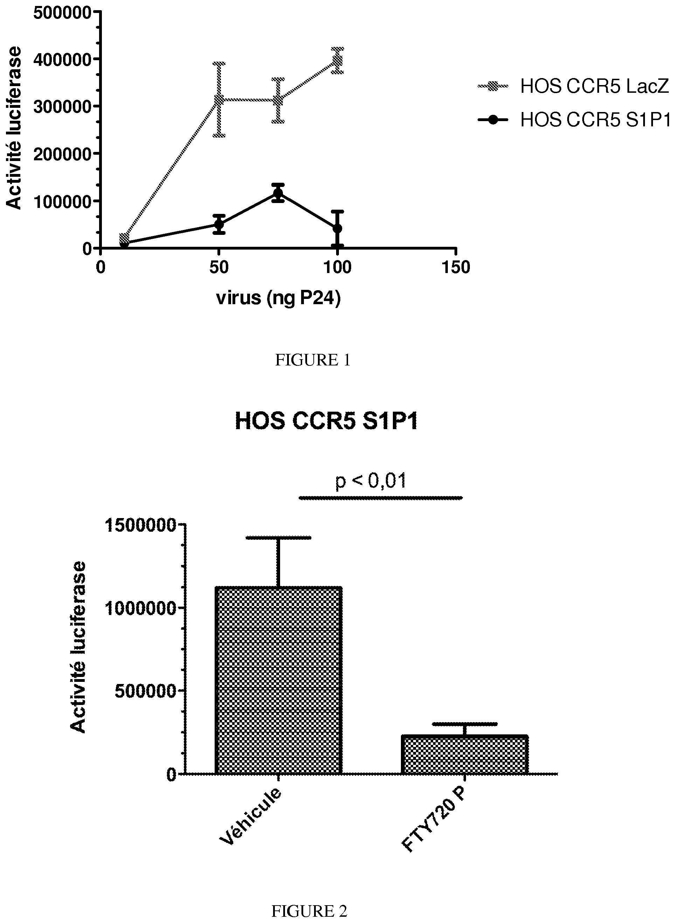

Ces deux lignées sont infectées par différentes doses de virus Ad8-luc et leur infectabilité est évaluée 72h après l'infection par mesure de l'activité luciférase (Figure 1).

On constate donc que la simple présence du récepteur S1PR1 inhibe l'infection de la lignée HOS par le VIH. Cet effet est très marqué, si l'on considère qu'il s'agit d'un virus « one-round » et qui donc n'effectue qu'un seul cycle de réplication. Ce résultat vient confirmer les résultats présentés précédemment sur la ligne HEK293 où le virus était utilisé à une dose unique.

### FTY720-P accentue l'inhibition de l'infection de la lignée HOS CCR5 S1PR1

Des cellules HOS CCR5 S1PR1 sont exposées pendant une heure au FTY720-P (33 µM) puis infectée comme précédemment (Figure 2).

Dans cette expérience, la pré-incubation avec FTY720-P des cellules exprimant le récepteur S1PR1 a diminué l'infection de 80% supplémentaire, en plus de l'inhibition constatée en présence du S1PR1 non stimulé.

### FTY720-P inhibe l'infection de la lignée HOS CCR5 en absence de S1PR1

Dans cette expérience, les inventeurs ont testé l'effet de différents agonistes des récepteurs S1PR sur l'infection par la souche Ad8-luc de la lignée HOS CCR5 qui n'exprime pas S1PR1.

Pour cela, des cellules HOS CCR5 lacZ ont été préincubées pendant 1 heure, soit avec un agoniste spécifique de S1PR1, le SEW2871 (33 µM), soit l'agoniste naturel de tous les récepteurs S1PR (S1PR1 à S1PR5), la sphingosine-1-phosphate (S1P, 33 µM), soit le FTY720-P (33 µM) qui est agoniste de S1PR1, S1PR3, S1PR4 et S1PR5 (Figure 3).

Il apparaît d'abord que le SEW2871 ne modifie pas significativement l'infection dans cette expérience, résultat attendu étant donné que cette lignée n'exprime pas le S1PR1.

Par contre, le S1P et le FTY720 diminuent l'infection mesurée d'environ 50%, et ceci de manière très significative (test de student, p <0,01). Il s'avère donc qu'outre le fait que la stimulation de S1PR1 diminue l'infection d'une lignée qui exprime ce récepteur à sa surface, que des agonistes des récepteurs S1PR2 à S1PR5 ont également un effet protecteur de l'infection.

### III. Inhibition de l'infection de cellules primaires lymphocytaires par le FTY720-P

Des cellules mononuclées du sang périphérique (PBMC) isolées à partir d'un donneur sain ont été préincubées en présence de FTY720-P à 1 µM ou de S1P à 1 µM, puis infectées par du virus Ad8-luc pendant 72h avant de mesurer l'activité luciférase. Les résultats montrent une inhibition d'environ 60% de l'infection par ces deux agonistes (Figure 4). L'expression du récepteur S1PR1 à la surface des PBMC n'a pas été détectée en cytométrie de flux (malgré la présence d'ARNm dans ces cellules).

Il est donc probable que l'effet de ces agonistes s'explique par la stimulation d'au moins un autre récepteur parmi les S 1 PR2, S 1 PR3, S 1 PR4, S1PR5.

Ce résultat confirme le fait que l'inhibition de l'infection par FTY720-P n'est pas seulement due à son effet sur S1PR1.

Pour confirmer cet effet sur les cellules primaires, des PBMC de donneurs sains ont été préincubées avec du FTY720-P à 1 µM pendant une heure, puis infectées avec du virus réplicatif Ad8. La quantité de virus produit dans le milieu de culture a été évaluée par mesure de la protéine virale p24 à 4, 7, 9 et 11 jours après l'infection (Figure 5).

On observe une nette inhibition de la production virale par les PBMC, qui atteint 77% en moyenne au 9ème jour.

Ainsi, les inventeurs ont démontré l'effet des agonistes des récepteurs de la sphingosine-1-phosphate à la fois sur des lignées cellulaires et sur des cellules primaires.

### IV. Test de l'efficacité de l'activité anti-VIH de FTY720 in vivo

Cette expérience permet de confirmer *in vivo* dans le modèle de souris SCID humanisées (hu-PBL-SCID) et infectées par le VIH-1, l'activité anti-VIH du FTY720 montrée préalablement *in vitro* sur des lignées ou des cellules primaires.

Deux groupes d'animaux sont comparés vis-à-vis de l'infection par le VIH ; un groupe contrôle de 8 animaux non traités et un groupe de 6 animaux traités avec le FTY720. L'expérience a été réalisée comme suit :
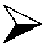 J-21: Réception des souris en animalerie A3 et adaptation d'une semaine à cet environnement.
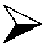 J-14: Reconstitution immunitaire des animaux par injection intra-péritonéale de PBMC humaines.
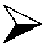 J-1: Evaluation de la greffe immunitaire et constitution de deux groupes de souris ayant une distribution de souris reconstituées comparable. Début du traitement. Un gavage avec du FTY720 à 0,3mg par kg et par jour ou avec le véhicule, puis gavage journalier pendant 15 jours.
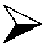 J0 : Infection des souris par le virus VIH-1 de type R5.
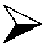 J6, 9, 12 : Dosage de la charge virale et sacrifice des animaux à J12.

La valeur moyenne des virémies pour chacun des 2 groupes est représentée dans la figure 6 (* : test de student, correction de Welch). On observe une diminution significative (p = 0,023) de l'infection des animaux traités. Cette expérience confirme donc l'effet anti-viral du FTY720 sur le VIH *in vivo,* dans le modèle de souris « humanisées ».

## Revendications

1. Composé qui est un agoniste d'au moins un récepteur choisi parmi les récepteurs S1PR1, S1PR2, S1PR3, S1PR4 et S1PR5 pour une utilisation dans une méthode de traitement d'une infection à VIH chez l'homme,
**caractérisé en ce que** ledit composé est FTY720 ou FTY720-P.

2. Composition pharmaceutique pour une utilisation dans une méthode de traitement d'une infection à VIH chez l'homme, ladite composition comprenant un composé tel que défini dans la revendication 1 et un véhicule pharmaceutiquement acceptable.

3. Composé destiné à être utilisé selon la revendication 1, ou composition destinée à être utilisée selon la revendication 2, ladite infection à VIH étant une infection à VIH-1.

4. Composé destiné à être utlisé selon la revendication 1, ou composition destinée à être utlisée selon la revendication 2, ladite infection à VIH étant une infection à VIH-2.

## Patentansprüche

1. Verbindung, die ein Agonist von mindestens einem Rezeptor ist, der ausgewählt ist aus den Rezeptoren S1PR1, S1PR2, S1PR3, S1PR4 und S1PR5, zur Verwendung in einem Verfahren zur Behandlung einer HIV-Infektion beim Menschen,
**dadurch gekennzeichnet, dass** die Verbindung FTY720 oder FTY720-P ist.

2. Pharmazeutische Zusammensetzung zur Verwendung in einem Verfahren zur Behandlung einer HIV-Infektion beim Menschen, wobei die Zusammensetzung eine wie in Anspruch 1 definierte Verbindung und einen pharmazeutisch annehmbaren Träger umfasst.

3. Verbindung zur Verwendung gemäß Anspruch 1, oder Zusammensetzung zur Verwendung gemäß Anspruch 2, wobei die HIV-Infektion eine HIV-1-Infektion ist.

4. Verbindung zur Verwendung gemäß Anspruch 1, oder Zusammensetzung zur Verwendung gemäß Anspruch 2, wobei die HIV-Infektion eine HIV-2-Infektion ist.

## Claims

1. A compound which is an agonist of at least one receptor selected from S1PR1, S1PR2, S1PR3, S1PR4 and S1PR5 receptors for use in a method for treating an HIV infection in humans, **characterized in that** said compound is FTY720 or FTY720-P.

2. A pharmaceutical composition for use in a method for treating an HIV infection in humans, said composition comprising a compound as defined in claim 1 and a pharmaceutically acceptable vehicle.

3. Compound for use according to claim 1, or composition for use according to claim 2, said HIV infection being an HIV-1 infection.

4. Compound for use according to claim 1 or composition for use according to claim 2, said HIV infection being an HIV-2 infection.


### SEQUENCE LISTING

<110> François, Vincent
<120> Inhibiteurs des infections à VIH et leurs utilisations
<130> BCT 110459 QT
<160> 6
<170> PatentIn version 3.3
<210> 1
   <211> 382
   <212> PRT
   <213> Homo sapiens
<400> 1
   Met Gly Pro Thr Ser Val Pro Leu Val Lys Ala His Arg Ser Ser Val
   1               5                   10                  15
   Ser Asp Tyr Val Asn Tyr Asp Ile Ile Val Arg His Tyr Asn Tyr Thr
               20                  25                  30
   Gly Lys Leu Asn Ile Ser Ala Asp Lys Glu Asn Ser Ile Lys Leu Thr
           35                  40                  45
   Ser Val Val Phe Ile Leu Ile Cys Cys Phe Ile Ile Leu Glu Asn Ile
       50                  55                  60
   Phe Val Leu Leu Thr Ile Trp Lys Thr Lys Lys Phe His Arg Pro Met
   65                  70                  75                  80
   Tyr Tyr Phe Ile Gly Asn Leu Ala Leu Ser Asp Leu Leu Ala Gly Val
                   85                  90                  95
   Ala Tyr Thr Ala Asn Leu Leu Leu Ser Gly Ala Thr Thr Tyr Lys Leu
               100                 105                 110
   Thr Pro Ala Gln Trp Phe Leu Arg Glu Gly Ser Met Phe Val Ala Leu
           115                 120                 125
   Ser Ala Ser Val Phe Ser Leu Leu Ala Ile Ala Ile Glu Arg Tyr Ile
       130                 135                 140
   Thr Met Leu Lys Met Lys Leu His Asn Gly Ser Asn Asn Phe Arg Leu
   145                 150                 155                 160
   Phe Leu Leu Ile Ser Ala Cys Trp Val Ile Ser Leu Ile Leu Gly Gly
                   165                 170                 175
   Leu Pro Ile Met Gly Trp Asn Cys Ile Ser Ala Leu Ser Ser Cys Ser
               180                 185                 190
   Thr Val Leu Pro Leu Tyr His Lys His Tyr Ile Leu Phe Cys Thr Thr
           195                 200                 205
   Val Phe Thr Leu Leu Leu Leu Ser Ile Val Ile Leu Tyr Cys Arg Ile
       210                 215                 220
   Tyr Ser Leu Val Arg Thr Arg Ser Arg Arg Leu Thr Phe Arg Lys Asn
   225                 230                 235                 240
   Ile Ser Lys Ala Ser Arg Ser Ser Glu Lys Ser Leu Ala Leu Leu Lys
                   245                 250                 255
   Thr Val Ile Ile Val Leu Ser Val Phe Ile Ala Cys Trp Ala Pro Leu
               260                 265                 270
   Phe Ile Leu Leu Leu Leu Asp Val Gly Cys Lys Val Lys Thr Cys Asp
           275                 280                 285
   Ile Leu Phe Arg Ala Glu Tyr Phe Leu Val Leu Ala Val Leu Asn Ser
       290                 295                 300
   Gly Thr Asn Pro Ile Ile Tyr Thr Leu Thr Asn Lys Glu Met Arg Arg
   305                 310                 315                 320
   Ala Phe Ile Arg Ile Met Ser Cys Cys Lys Cys Pro Ser Gly Asp Ser
                   325                 330                 335
   Ala Gly Lys Phe Lys Arg Pro Ile Ile Ala Gly Met Glu Phe Ser Arg
               340                 345                 350
   Ser Lys Ser Asp Asn Ser Ser His Pro Gln Lys Asp Glu Gly Asp Asn
           355                 360                 365
   Pro Glu Thr Ile Met Ser Ser Gly Asn Val Asn Ser Ser Ser
       370                 375                 380
<210> 2
   <211> 353
   <212> PRT
   <213> Homo sapiens
<400> 2
   Met Gly Ser Leu Tyr Ser Glu Tyr Leu Asn Pro Asn Lys Val Gln Glu
   1               5                   10                  15
   His Tyr Asn Tyr Thr Lys Glu Thr Leu Glu Thr Gln Glu Thr Thr Ser
               20                  25                  30
   Arg Gln Val Ala Ser Ala Phe Ile Val Ile Leu Cys Cys Ala Ile Val
           35                  40                  45
   Val Glu Asn Leu Leu Val Leu Ile Ala Val Ala Arg Asn Ser Lys Phe
       50                  55                  60
   His Ser Ala Met Tyr Leu Phe Leu Gly Asn Leu Ala Ala Ser Asp Leu
   65                  70                  75                  80
   Leu Ala Gly Val Ala Phe Val Ala Asn Thr Leu Leu Ser Gly Ser Val
                   85                  90                  95
   Thr Leu Arg Leu Thr Pro Val Gln Trp Phe Ala Arg Glu Gly Ser Ala
               100                 105                 110
   Phe Ile Thr Leu Ser Ala Ser Val Phe Ser Leu Leu Ala Ile Ala Ile
           115                 120                 125
   Glu Arg His Val Ala Ile Ala Lys Val Lys Leu Tyr Gly Ser Asp Lys
       130                 135                 140
   Ser Cys Arg Met Leu Leu Leu Ile Gly Ala Ser Trp Leu Ile Ser Leu
   145                 150                 155                 160
   Val Leu Gly Gly Leu Pro Ile Leu Gly Trp Asn Cys Leu Gly His Leu
                   165                 170                 175
   Glu Ala Cys Ser Thr Val Leu Pro Leu Tyr Ala Lys His Tyr Val Leu
               180                 185                 190
   Cys Val Val Thr Ile Phe Ser Ile Ile Leu Leu Ala Ile Val Ala Leu
           195                 200                 205
   Tyr Val Arg Ile Tyr Cys Val Val Arg Ser Ser His Ala Asp Met Ala
       210                 215                 220
   Ala Pro Gln Thr Leu Ala Leu Leu Lys Thr Val Thr Ile Val Leu Gly
   225                 230                 235                 240
   Val Phe Ile Val Cys Trp Leu Pro Ala Phe Ser Ile Leu Leu Leu Asp
                   245                 250                 255
   Tyr Ala Cys Pro Val His Ser Cys Pro Ile Leu Tyr Lys Ala His Tyr
               260                 265                 270
   Phe Phe Ala Val Ser Thr Leu Asn Ser Leu Leu Asn Pro Val Ile Tyr
   275 280 285
   Thr Trp Arg Ser Arg Asp Leu Arg Arg Glu Val Leu Arg Pro Leu Gln
   290 295 300
   Cys Trp Arg Pro Gly Val Gly Val Gln Gly Arg Arg Arg Gly Gly Thr
   305 310 315 320
   Pro Gly His His Leu Leu Pro Leu Arg Ser Ser Ser Ser Leu Glu Arg
   325 330 335
   Gly Met His Met Pro Thr Ser Pro Thr Phe Leu Glu Gly Asn Thr Val
   340 345 350
   Val
<210> 3
   <211> 378
   <212> PRT
   <213> Homo sapiens
<400> 3
   Met Ala Thr Ala Leu Pro Pro Arg Leu Gln Pro Val Arg Gly Asn Glu
   1 5 10 15
   Thr Leu Arg Glu His Tyr Gln Tyr Val Gly Lys Leu Ala Gly Arg Leu
   20 25 30
   Lys Glu Ala Ser Glu Gly Ser Thr Leu Thr Thr Val Leu Phe Leu Val
   35 40 45
   Ile Cys Ser Phe Ile Val Leu Glu Asn Leu Met Val Leu Ile Ala Ile
   50 55 60
   Trp Lys Asn Asn Lys Phe His Asn Arg Met Tyr Phe Phe Ile Gly Asn
   65 70 75 80
   Leu Ala Leu Cys Asp Leu Leu Ala Gly Ile Ala Tyr Lys Val Asn Ile
   85 90 95
   Leu Met Ser Gly Lys Lys Thr Phe Ser Leu Ser Pro Thr Val Trp Phe
   100 105 110
   Leu Arg Glu Gly Ser Met Phe Val Ala Leu Gly Ala Ser Thr Cys Ser
   115 120 125
   Leu Leu Ala Ile Ala Ile Glu Arg His Leu Thr Met Ile Lys Met Arg
       130                 135                 140
   Pro Tyr Asp Ala Asn Lys Arg His Arg Val Phe Leu Leu Ile Gly Met
   145                 150                 155                 160
   Cys Trp Leu Ile Ala Phe Thr Leu Gly Ala Leu Pro Ile Leu Gly Trp
                   165                 170                 175
   Asn Cys Leu His Asn Leu Pro Asp Cys Ser Thr Ile Leu Pro Leu Tyr
               180                 185                 190
   Ser Lys Lys Tyr Ile Ala Phe Cys Ile Ser Ile Phe Thr Ala Ile Leu
           195                 200                 205
   Val Thr Ile Val Ile Leu Tyr Ala Arg Ile Tyr Phe Leu Val Lys Ser
       210                 215                 220
   Ser Ser Arg Lys Val Ala Asn His Asn Asn Ser Glu Arg Ser Met Ala
   225                 230                 235                 240
   Leu Leu Arg Thr Val Val Ile Val Val Ser Val Phe Ile Ala Cys Trp
                   245                 250                 255
   Ser Pro Leu Phe Ile Leu Phe Leu Ile Asp Val Ala Cys Arg Val Gln
               260                 265                 270
   Ala Cys Pro Ile Leu Phe Lys Ala Gln Trp Phe Ile Val Leu Ala Val
           275                 280                 285
   Leu Asn Ser Ala Met Asn Pro Val Ile Tyr Thr Leu Ala Ser Lys Glu
       290                 295                 300
   Met Arg Arg Ala Phe Phe Arg Leu Val Cys Asn Cys Leu Val Arg Gly
   305                 310                 315                 320
   Arg Gly Ala Arg Ala Ser Pro Ile Gln Pro Ala Leu Asp Pro Ser Arg
                   325                 330                 335
   Ser Lys Ser Ser Ser Ser Asn Asn Ser Ser His Ser Pro Lys Val Lys
               340                 345                 350
   Glu Asp Leu Pro His Thr Ala Pro Ser Ser Cys Ile Met Asp Lys Asn
           355                 360                 365
   Ala Ala Leu Gln Asn Gly Ile Phe Cys Asn
       370                 375
<210> 4
   <211> 384
   <212> PRT
   <213> Homo sapiens
<400> 4
   Met Asn Ala Thr Gly Thr Pro Val Ala Pro Glu Ser Cys Gln Gln Leu
   1               5                   10                  15
   Ala Ala Gly Gly His Ser Arg Leu Ile Val Leu His Tyr Asn His Ser
               20                  25                  30
   Gly Arg Leu Ala Gly Arg Gly Gly Pro Glu Asp Gly Gly Leu Gly Ala
           35                  40                  45
   Leu Arg Gly Leu Ser Val Ala Ala Ser Cys Leu Val Val Leu Glu Asn
       50                  55                  60
   Leu Leu Val Leu Ala Ala Ile Thr Ser His Met Arg Ser Arg Arg Trp
   65                  70                  75                  80
   Val Tyr Tyr Cys Leu Val Asn Ile Thr Leu Ser Asp Leu Leu Thr Gly
                   85                  90                  95
   Ala Ala Tyr Leu Ala Asn Val Leu Leu Ser Gly Ala Arg Thr Phe Arg
               100                 105                 110
   Leu Ala Pro Ala Gln Trp Phe Leu Arg Glu Gly Leu Leu Phe Thr Ala
           115                 120                 125
   Leu Ala Ala Ser Thr Phe Ser Leu Leu Phe Thr Ala Gly Glu Arg Phe
       130                 135                 140
   Ala Thr Met Val Arg Pro Val Ala Glu Ser Gly Ala Thr Lys Thr Ser
   145                 150                 155                 160
   Arg Val Tyr Gly Phe Ile Gly Leu Cys Trp Leu Leu Ala Ala Leu Leu
                   165                 170                 175
   Gly Met Leu Pro Leu Leu Gly Trp Asn Cys Leu Cys Ala Phe Asp Arg
               180                 185                 190
   Cys Ser Ser Leu Leu Pro Leu Tyr Ser Lys Arg Tyr Ile Leu Phe Cys
           195                 200                 205
   Leu Val Ile Phe Ala Gly Val Leu Ala Thr Ile Met Gly Leu Tyr Gly
       210                 215                 220
   Ala Ile Phe Arg Leu Val Gln Ala Ser Gly Gln Lys Ala Pro Arg Pro
   225                 230                 235                 240
   Ala Ala Arg Arg Lys Ala Arg Arg Leu Leu Lys Thr Val Leu Met Ile
                   245                 250                 255
   Leu Leu Ala Phe Leu Val Cys Trp Gly Pro Leu Phe Gly Leu Leu Leu
               260                 265                 270
   Ala Asp Val Phe Gly Ser Asn Leu Trp Ala Gln Glu Tyr Leu Arg Gly
           275                 280                 285
   Met Asp Trp Ile Leu Ala Leu Ala Val Leu Asn Ser Ala Val Asn Pro
       290                 295                 300
   Ile Ile Tyr Ser Phe Arg Ser Arg Glu Val Cys Arg Ala Val Leu Ser
   305                 310                 315                 320
   Phe Leu Cys Cys Gly Cys Leu Arg Leu Gly Met Arg Gly Pro Gly Asp
                   325                 330                 335
   Cys Leu Ala Arg Ala Val Glu Ala His Ser Gly Ala Ser Thr Thr Asp
               340                 345                 350
   Ser Ser Leu Arg Pro Arg Asp Ser Phe Arg Gly Ser Arg Ser Leu Ser
           355                 360                 365
   Phe Arg Met Arg Glu Pro Leu Ser Ser Ile Ser Ser Val Arg Ser Ile
       370                 375                 380
<210> 5
   <211> 310
   <212> PRT
   <213> Homo sapiens
<400> 5
   Met Glu Ser Gly Leu Leu Arg Pro Ala Pro Val Ser Glu Val Ile Val
   1               5                   10                  15
   Leu His Tyr Asn Tyr Thr Gly Lys Leu Arg Gly Ala Arg Tyr Gln Pro
               20                  25                  30
   Gly Ala Gly Leu Arg Ala Asp Ala Val Val Cys Leu Ala Val Cys Ala
           35                  40                  45
   Phe Ile Val Leu Glu Asn Leu Ala Val Leu Leu Val Leu Gly Arg His
       50                  55                  60
   Pro Arg Phe His Ala Pro Met Phe Leu Leu Leu Gly Ser Leu Thr Leu
   65                  70                  75                  80
   Ser Asp Leu Leu Ala Gly Ala Ala Tyr Ala Ala Asn Ile Leu Leu Ser
                   85                  90                  95
   Gly Pro Leu Thr Leu Lys Leu Ser Pro Ala Leu Trp Phe Ala Arg Glu
               100                 105                 110
   Gly Gly Val Phe Val Ala Leu Thr Ala Ser Val Leu Ser Leu Leu Ala
           115                 120                 125
   Ile Ala Leu Glu Arg Ser Leu Thr Met Ala Arg Arg Gly Pro Ala Pro
       130                 135                 140
   Val Ser Ser Arg Gly Arg Thr Leu Ala Met Ala Ala Ala Ala Trp Gly
   145                 150                 155                 160
   Val Ser Leu Leu Leu Gly Leu Leu Pro Ala Leu Gly Trp Asn Cys Leu
                   165                 170                 175
   Gly Arg Leu Asp Ala Cys Ser Thr Val Leu Pro Leu Tyr Ala Lys Ala
               180                 185                 190
   Tyr Val Leu Phe Cys Val Leu Ala Phe Val Gly Ile Leu Ala Ala Ile
           195                 200                 205
   Cys Ala Leu Tyr Ala Leu Ala Gly Leu Ala Ala His Ala Gln Arg Gly
       210                 215                 220
   Ala Pro Gly Leu Cys Gly Met Leu Gly Pro Pro His Pro Ala Ala Val
   225                 230                 235                 240
   Ala Arg Arg Gly Val Pro Gly Ala His Leu Ser Cys Thr Pro Ala Gly
                   245                 250                 255
   Arg Ser Leu Pro Gly Thr Gly His Gly Gln Leu Thr Ser Glu Pro His
               260                 265                 270
   His Leu His Ala His Gln Pro Arg Pro Ala Pro Arg Ala Pro Ala Pro
           275                 280                 285
   Gly Leu Leu Arg Thr Pro Leu Leu Arg Gln Arg Pro Glu Trp Leu Pro
       290                 295                 300
   Ala Val Gly Glu Arg Gly
   305 310
<210> 6
   <211> 398
   <212> PRT
   <213> Homo sapiens
<400> 6
   Met Glu Ser Gly Leu Leu Arg Pro Ala Pro Val Ser Glu Val Ile Val
   1               5                   10                  15
   Leu His Tyr Asn Tyr Thr Gly Lys Leu Arg Gly Ala Arg Tyr Gln Pro
               20                  25                  30
   Gly Ala Gly Leu Arg Ala Asp Ala Val Val Cys Leu Ala Val Cys Ala
           35                  40                  45
   Phe Ile Val Leu Glu Asn Leu Ala Val Leu Leu Val Leu Gly Arg His
       50                  55                  60
   Pro Arg Phe His Ala Pro Met Phe Leu Leu Leu Gly Ser Leu Thr Leu
   65                  70                  75                  80
   Ser Asp Leu Leu Ala Gly Ala Ala Tyr Ala Ala Asn Ile Leu Leu Ser
                   85                  90                  95
   Gly Pro Leu Thr Leu Lys Leu Ser Pro Ala Leu Trp Phe Ala Arg Glu
               100                 105                 110
   Gly Gly Val Phe Val Ala Leu Thr Ala Ser Val Leu Ser Leu Leu Ala
           115                 120                 125
   Ile Ala Leu Glu Arg Ser Leu Thr Met Ala Arg Arg Gly Pro Ala Pro
       130                 135                 140
   Val Ser Ser Arg Gly Arg Thr Leu Ala Met Ala Ala Ala Ala Trp Gly
   145                 150                 155                 160
   Val Ser Leu Leu Leu Gly Leu Leu Pro Ala Leu Gly Trp Asn Cys Leu
                   165                 170                 175
   Gly Arg Leu Asp Ala Cys Ser Thr Val Leu Pro Leu Tyr Ala Lys Ala
               180                 185                 190
   Tyr Val Leu Phe Cys Val Leu Ala Phe Val Gly Ile Leu Ala Ala Ile
           195                 200                 205
   Cys Ala Leu Tyr Ala Arg Ile Tyr Cys Gln Val Arg Ala Asn Ala Arg
       210                 215                 220
   Arg Leu Pro Ala Arg Pro Gly Thr Ala Gly Thr Thr Ser Thr Arg Ala
   225                 230                 235                 240
   Arg Arg Lys Pro Arg Ser Leu Ala Leu Leu Arg Thr Leu Ser Val Val
                   245                 250                 255
   Leu Leu Ala Phe Val Ala Cys Trp Gly Pro Leu Phe Leu Leu Leu Leu
               260                 265                 270
   Leu Asp Val Ala Cys Pro Ala Arg Thr Cys Pro Val Leu Leu Gln Ala
           275                 280                 285
   Asp Pro Phe Leu Gly Leu Ala Met Ala Asn Ser Leu Leu Asn Pro Ile
       290                 295                 300
   Ile Tyr Thr Leu Thr Asn Arg Asp Leu Arg His Ala Leu Leu Arg Leu
   305                 310                 315                 320
   Val Cys Cys Gly Arg His Ser Cys Gly Arg Asp Pro Ser Gly Ser Gln
                   325                 330                 335
   Gln Ser Ala Ser Ala Ala Glu Ala Ser Gly Gly Leu Arg Arg Cys Leu
               340                 345                 350
   Pro Pro Gly Leu Asp Gly Ser Phe Ser Gly Ser Glu Arg Ser Ser Pro
           355                 360                 365
   Gln Arg Asp Gly Leu Asp Thr Ser Gly Ser Thr Gly Ser Pro Gly Ala
       370                 375                 380
   Pro Thr Ala Ala Arg Thr Leu Val Ser Glu Pro Ala Ala Asp
   385                 390                 395